Europäisches Patentamt

(19) European Patent Office

Office européen des brevets

(11) Numéro de publication : **0 014 136**
**B1**

(12) **FASCICULE DE BREVET EUROPÉEN**

(45) Date de publication du fascicule du brevet :
**09.09.81**

(21) Numéro de dépôt : **80400078.4**

(22) Date de dépôt : **18.01.80**

(51) Int. Cl.³ : **C 02 F 3/28**, C 02 F 11/04

(54) Fermenteur décanteur anaérobie pour l'épuration d'eaux résiduaires de sucrerie avec récupération de méthane combustible.

(30) Priorité : **24.01.79 FR 7901775**

(43) Date de publication de la demande :
**06.08.80 (Bulletin 80/16)**

(45) Mention de la délivrance du brevet :
**09.09.81 Bulletin 81/36**

(84) Etats contractants désignés :
**AT BE CH DE GB IT LU NL SE**

(56) Documents cités :
**GB - A - 837 561**
**US - A - 4 060 175**

(73) Titulaire : **SYNDICAT NATIONAL DES FABRICANTS DE SUCRE DE FRANCE**
**23, avenue d'Iéna**
**F-75016 Paris (FR)**

(72) Inventeur : **Lescure, Jean-Pierre**
**17, avenue Robert Schumann**
**F-59370 Mons-en-Baroeul (FR)**

(74) Mandataire : **CABINET BONNET-THIRION**
**95 Boulevard Beaumarchais**
**F-75003 Paris (FR)**

Imprimerie Jouve, 17, rue du Louvre, 75001 Paris, France

**0 014 136**

### Fermenteur décanteur anaérobie pour l'épuration d'eaux résiduaires de sucrerie avec récupération de méthane combustible

L'invention se rapporte à un fermenteur décanteur destiné à l'épuration d'eaux résiduaires notamment de sucrerie avec récupération des gaz de fermentation constitués en majeure partie de méthane combustible.

L'épuration classique par lagunage des eaux résiduaires de sucrerie contenant des sucres divers, qui sont détruits par fermentation au cours du lagunage nécessite des bassins de grande étendue, est source d'odeurs désagréables pour le voisinage, ne permet pas un contrôle précis du taux d'épuration effectif, et enfin conduit à laisser se disperser dans le milieu ambiant l'énergie contenue dans les produits sucrés.

La Demanderesse a effectué des études sur l'épuration par fermentation des eaux résiduaires sous l'action de microorganismes anaérobies mésophiles (actifs dans une gamme de température autour de 35 °C), le terme de ce type de fermentation étant principalement le méthane et le gaz carbonique, ce dernier étant en grande partie retenu en combinaison carbonatée, tandis que le méthane qui se dégage constitue un combustible utilisable. En fait la fermentation se produit en deux temps, des micro-organismes acidifiants transformant dans le premier temps les sucres en acides organiques, tandis que des micro-organismes anaérobies stricts, mésophiles décomposent les acides en méthane, gaz carbonique et eau.

Les études de la Demanderesse ont porté sur des essais en laboratoire, puis à des essais sur fermenteurs pilotes. Un premier pilote, comportant un bassin de 90 m$^3$ à la sucrerie d'Escaudœuvres a été suivi d'un second, avec un bassin de 1 580 m$^3$ à la sucrerie raffinerie de Vauciennes. On trouvera l'exposé des travaux et la description des installations dans la revue Sucrerie Française : « La dépollution des eaux » par J.P. Lescure et P. Bourlet, mars 1977, pages 103-109 ; « Nouvelles perspectives pour le traitement des eaux en sucrerie ; la fermentation méthanique mésophile », P. Devillers, J.P. Lescure et P. Bourlet, avril 1977, pages 173-183 ; et « Traitement des eaux résiduaires par fermentation méthanique mésophile » J.P. Lescure, P. Bourlet, mars 1978, pages 107-114. Dans les installations décrites, la fermentation s'effectuait vers 35 °C par passage de l'influent dans un réchauffement, d'où cet influent était amené par une tuyauterie au fond et près du centre du bassin, où il était brassé par un agitateur rotatif. Une bâche en caoutchouc-butyle armé était ancrée par sa périphérie en immersion dans le bassin et recouvrait la zone de fermentation en sorte de former poche de collection pour le méthane dégagé, qui était évacué par une tubulure, fixée à la partie supérieure de la poche de bâche. Cette disposition, qui a permis une mise au point poussée du procédé de fermentation, présentant toutefois des inconvénients affectant la sécurité d'exploitation et la fiabilité de l'installation. En effet la bâche n'était pas tendue et émergeait partiellement. L'agitation des eaux résiduaires sous la bâche se transmettait à celle-ci, qui risquait des déchirures prématurées. Des fuites à travers la bâche, par des amorces de déchirures, créaient un danger d'explosion ou d'incendie de la poche gonflée par le gaz.

Pour remédier à ces inconvénients l'invention propose un fermenteur décanteur anaérobie, destiné à l'épuration d'eaux résiduaires notamment de l'industrie du sucre avec récupération des gaz de fermentation constitués en majeure partie de méthane combustible, et comprenant un bassin creusé dans le sol avec une peau d'étanchéité, en forme de pyramide tronquée avec des parois s'évasant vers le haut et un fond sensiblement plat, et adapté à être sensiblement rempli d'eau résiduaire à épurer, vers le centre du bassin un agitateur rotatif à arbre vertical avec un impulseur au voisinage du fond, une bâche souple étendue dans la zone centrale du bassin, ancrée par sa périphérie sensiblement à mi-hauteur des parois et formant poche de collection des gaz de fermentation avec une tubulure d'évacuation des gaz de fermentation en point haut, et une tuyauterie d'amenée d'eaux résiduaires passant par un réchauffeur et débouchant au voisinage de l'impulseur, fermenteur caractérisé en ce qu'une cloche collectrice métallique, avec des bords immergés et traversée axialement par l'arbre de l'agitateur, se raccorde par ses bords à une ouverture centrale de la bâche, celle-ci étant tendue entre ouverture centrale et points d'ancrage périphériques.

La cloche collectrice, métallique, émergeant seule du bassin, les gaz de fermentation ne sont présents en quantité notable, à l'état non dispersé dans les eaux résiduaires, que dans la cloche et la tubulure d'évacuation. Le danger de fuites de gaz combustible est donc pratiquement éliminé. La bâche, tendue entre la cloche et les points d'ancrage, n'est pas susceptible de mouvements de grande ampleur ; en outre les mouvements de la bâche sont amortis par la présence d'eau sur ses deux faces. Les risques de déchirures sont donc très réduits.

De préférence la cloche comporte une chambre collectrice ouverte vers le bas et limitée latéralement par des bords intérieur et extérieur, ce dernier formant raccordement avec l'ouverture centrale de la bâche, tandis que le bord intérieur entoure l'axe d'agitateur en définissant avec un voile inférieur une cavité fermée. On réduit ainsi des fuites de gaz le long de l'axe d'agitateur.

De préférence le bord extérieur de cloche est entouré d'une goulotte annulaire ouverte vers le haut, avec un rebord périphérique horizontal formant lame déversante et une canalisation d'évacuation d'eau résiduaire épurée. Comme le niveau d'eau résiduaire dans le bassin est déterminé par le rebord formant lame déversante franchie par l'eau résiduaire épurée qui s'évacue par la canalisation, la profondeur d'immersion des bords de cloche est maintenue constante.

Selon une disposition préférée la périphérie de la bâche est raidie par des tiges passant par des

2

**0 014 136**

ourlets et rattachées par des tendeurs aux points d'ancrage, ces derniers étant scellés dans un cadre formé par des poutres étendues horizontalement sensiblement à mi-hauteur du bassin. On assure ainsi une bonne rigidité des bords de bâche sans que celle-ci soit soumise à des efforts localisés excessifs. Le cadre en poutre assure par son poids et sa structure une bonne tenue des points d'ancrage, sans qu'il soit nécessaire de les planter dans le sol sousjacent en perçant la peau d'étanchéité.

Les caractéristiques et avantages de l'invention ressortiront d'ailleurs de la description qui va suivre, à titre d'exemple, en référence aux dessins annexés dans lesquels :

la figure 1 représente en coupe une vue générale d'un fermenteur selon l'invention ;

la figure 2 représente plus en détail une cloche collectrice ;

la figure 3 est une perspective schématique d'une bâche de collection ;

la figure 4 est une vue de détail de la disposition d'ancrage périphérique de la bâche.

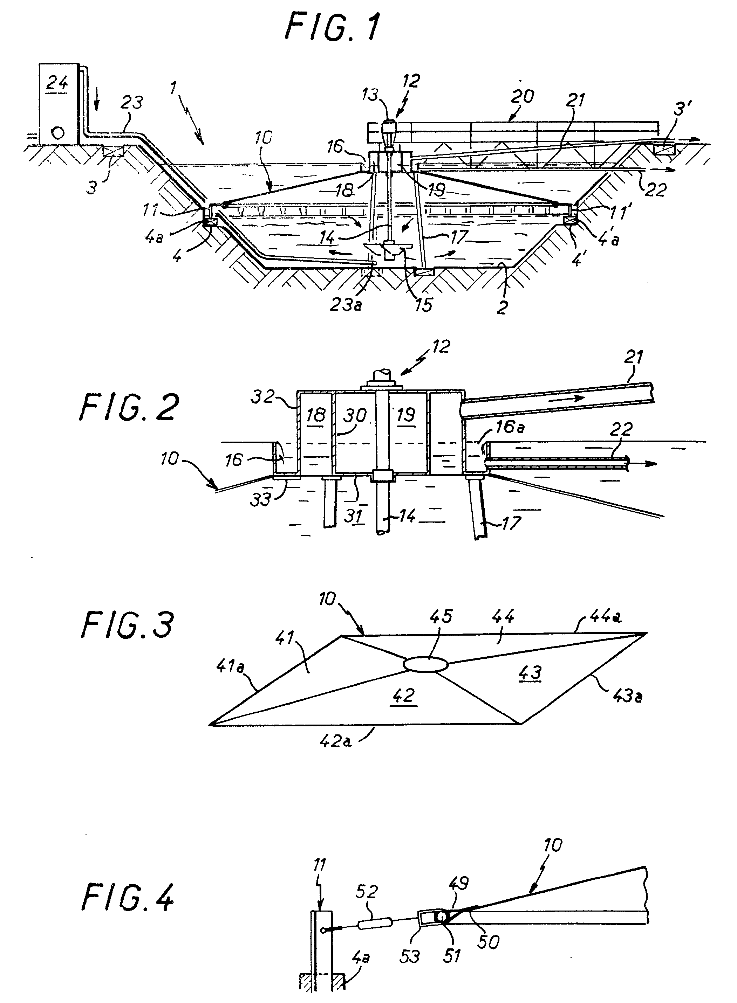

Selon la forme de réalisation choisie et représentée figure 1, un fermenteur décanteur comporte, creusé dans le sol, un bassin 1 dans son ensemble en forme générale de tronc de pyramide droite à base carrée, avec des bords s'évasant vers le haut et inclinés à environ 45°. Une peau d'étanchéité 2, formée de manière connue par assemblage de feuilles de plastique, est fixée à sa périphérie par enfouissage dans des fossés 3,3' et chargée par des blocs de maçonnerie. A mi-hauteur des bords inclinés sont ménagées des bermes horizontales 4,4', épousées par la peau d'étanchéité. Sur ces bermes 4,4' sont posées des poutres en béton 4a, 4'a qui forment un cadre dans lequel sont scellés des pieux ou points d'ancrage 11, 11', pour une bâche 10 en forme de pyramide à base carrée. Au sommet de la bâche 10, celle-ci se raccorde à une cloche collectrice 12 dans son ensemble émergeant partiellement, et qui porte en son centre un moteur 13 pour un agitateur rotatif à arbre 14 vertical terminé au voisinage du fond du bassin par un impulseur hélicoïdal 15. La cloche 12 est tenue sur le fond du bassin par un support tripode 17, et est reliée au pourtour du bassin par une passerelle 20, qui forme support pour une tubulure d'évacuation de gaz de fermentation 21, qui débouche de la chambre annulaire 18, ouverte vers le bas de la cloche 12, et pour une canalisation 22 d'évacuation des eaux résiduaires épurées qui part de la goulotte périphérique 16. Les eaux résiduaires à épurer, ou influents, sont introduits dans le bassin 1 par une tuyauterie 23 dont l'extrémité 23a débouche au voisinage du fond du bassin, à proximité de l'impulseur 15 ; après avoir traversé un réchauffeur 24, destiné à porter la température des influents au voisinage de 35 °C, température favorable à l'activité de micro-organismes anaérobies mésophiles.

On verra mieux figure 2 la disposition de la cloche 12, traversée verticalement par l'arbre 14 d'agitateur. La cloche se compose d'une chambre annulaire 18, limitée par des bords cylindriques intérieur 30 et extérieur 32, coaxiaux à l'arbre 14, et ouverte vers le bas. Le bord intérieur 30 porte un voile 31 à sa partie inférieure, pour définir une cavité fermée 19 traversée par l'arbre 14. Entourant le bord extérieur 30, la goulotte annulaire 16 est munie d'un rebord périphérique 16a qui forme lame déversante pour les eaux résiduaires épurées, ou effluents, en sorte de définir le niveau dans le bassin en assurant que les bords 30 et 32 sont immergés en permanence. La bâche 10 est serrée entre la base de la goulotte 16 et une couronne circulaire 33 qui termine le support tripode 17. On aura compris que les gaz de fermentation sous la bâche 10 viennent se rassembler dans la chambre 18 pour être évacués par la tubulure 21, sans pénétrer notablement dans la cavité 19, le voile noyé 31 faisant obstacle au passage des gaz.

Comme on le voit figure 3, la bâche 10 est formée de quatre pans triangulaires 41, 42, 43 et 44, convergeant vers l'ouverture circulaire sommitale 45 de raccordement à la cloche collectrice. Les bases 41a, 42a, 43a et 44a des pans sont raidies par des tiges pour former un carré.

La figure 4 montre plus en détail comment sont raidis et ancrés les bords de bâche 10. Le bord de bâche forme un ourlet 49, par rabattement et fixation de la bordure 50 sur la bâche 10. Une tige (tube métallique) 51 est enfilée dans l'ourlet 49. L'ancrage sur le pieu 11, scellé dans la poutre 4a est obtenu par un tendeur 52, du genre écrou long à pas inversé prenant sur deux tiges filetées, la tige située du côté de la bâche 10 se terminant par un étrier 53 qui chevauche la tige 51. Le vissage contrôlé des écrous de tendeurs permet de tendre régulièrement la bâche sur tout son pourtour, les efforts de tension des tendeurs 52 étant transmis uniformément à la bâche par l'intermédiaire de la tige de raidissement 51 et l'ourlet 49.

Dans un exemple de réalisation, le bassin, avec une peau étanche en chlorure de polyvinyle armé, présentait une ouverture, au niveau du sol, de 22 mètres de côté et une profondeur de 5 mètres, avec des pentes de parois de 45°. L'agitateur était un hélicomélangeur (SEMHM 2 500) à vitesse variable, permettant, à des régimes compris entre 3,2 et 22 tours par minute, des débits de circulation de 3 000 à 17 600 m$^3$/h, le moteur ayant une puissance de 7 360 W (10 CV).

Les influents introduits sous la bâche sont éventuellement additionnés de produits nutritifs azotés ou phosphorés en sorte d'obtenir un équilibre C/N/P~300/5/1. Le pH est maintenu par addition de chaux et d'écume (CaCO$_3$) de défécation de jus. Après fermentation les eaux résiduaires passent autour de la périphérie de la bâche pour venir se clarifier par décantation dans la partie du bassin située au-dessus de la bâche (surface de décantation 400 m$^2$ environ). Les boues sont évacuées de façon discontinue à l'aide d'une pompe immergée, tandis que les effluents clarifiés passent dans la goulotte et sont évacués au fur et à mesure de l'introduction d'influent.

Pour rendre compte des résultats d'essais on rappellera la signification des abréviations suivantes :

COT teneur en carbone organique total ;

3

DCO demande chimique en oxygène ;
DBO$_5$ demande biologique en oxygène, jusqu'au 5$^e$ jour.

Résultats d'épuration

| Semaine | Débit moyen m³/h | DCO enlevée Kg/j | Charge volumique Kg DCO/m³/j | Production de gaz m³/j | Rendement % |
|---|---|---|---|---|---|
| 43$^e$ | 27,0 | 2430 | 3,04 | 490 | 83 |
| 45$^e$ | 37,6 | 5022 | 6,28 | 960 | 83 |
| 47$^e$ | 31,9 | 3926 | 4,91 | 550 | 88 |
| 48$^e$ | 30 | 3000 | 3,75 | 518 | 70 |
| 49$^e$ | 18,3 | 2020 | 2,53 | 700 | 77 |
| 50$^e$ | 19,5 | 2915 | 3,64 | 706 | 97 |

Analyses

| Influent | minimum | moyenne | maximum |
|---|---|---|---|
| COT mg/l (C) | 1145 | 1956 | 2280 |
| DCO mg/l (O) | — | 5896 | 6900 |
| DBO$_5$ mg/l (O) | — | 3983 | 4920 |
| Azote Kjeldahl mg/l (N) | 37 | 49,7 | 62 |
| Phosphore total mg/l (P) | 6,3 | 8,4 | 9,9 |
| Soufre total | 0,4 | 6,5 | 19,3 |

| Effluent | | | |
|---|---|---|---|
| COT mg/l | 62 | 414 | 807 |
| DCO mg/l | 340 | 1107 | 2400 |
| DBO$_5$ mg/l | 95 | 720 | 1600 |
| N Kjeldahl mg/l | 14 | 36,7 | 41 |
| P total mg/l | 2,5 | 4,3 | 6,7 |
| S total mg/l | 0 | 3,0 | 7,5 |

| Gaz % | | | |
|---|---|---|---|
| $CO_2$ | 9,96 | 12,30 | 15,8 |
| $H_2$ | traces | 0,22 | 0,42 |
| $O_2$ | 0,8 | 1,11 | 1,76 |
| $N_2$ | 3,18 | 3,95 | 6,14 |
| $CH_4$ | 80,00 | 82,41 | 85,95 |

En recueillant 700 m³/j de gaz à 82,5 % de méthane, soit 420 kg de méthane par jour, on dispose d'une énergie calorifique d'environ $20,93 \times 10^9$ J (5 000 thermies), soit près de 5 800 kWh tandis que l'épuration des eaux résiduaires atteint un taux intéressant.

Bien entendu de l'énergie calorifique récupérable il faut déduire l'énergie nécessaire pour le chauffage de l'influent, mais le bilan reste positif, et peut être amélioré en faisant passer à contre-courant effluent et influent dans un échangeur de chaleur, en sorte que les pertes d'énergie de chauffage se limitent aux pertes en surface du bassin. Ces pertes croissent d'ailleurs en valeur absolue plus lentement que la capacité du bassin.

Bien que le fermenteur décanteur décrit ait été mis au point dans le cadre d'industries sucrières, qui produisent de grandes quantités d'effluents fermentescibles qui nécessitent une épuration avant rejet dans l'environnement, il est clair qu'il n'y a pas de lien de nécessité entre les structures décrites et l'origine des effluents fermentescibles, de sorte que le fermenteur décanteur est utilisable pour le traitement d'effluents de toute origine, du moment que ces effluents sont susceptibles de fermentation anaérobie sous l'action de micro-organismes spécifiques, avec du méthane pour terme ultime.

En effet, le méthane est le terme ultime principal de toute dégradation réductrice de matières organiques, et les micro-organismes anaérobies se sélectionnent d'eux-mêmes dans les conditions où leur activité est optimale, ces conditions comprenant le milieu nutritif (effluents éventuellement corrigés), la température, et une dispersion suffisante des micro-organismes dans l'effluent. De ce point de vue les dispositions prévues du fermenteur décanteur conviennent d'une façon générale, et se prêtent aux ajustements particuliers nécessaires. Par ailleurs la structure de la poche de collection totalement immergée convient pour recueillir tout gaz combustible formé au sein d'un liquide agité remplissant un bassin dans des conditions de sécurité et de fiabilité, pour un coût d'installation relativement réduit, quel que soit le processus de formation du gaz combustible. Mais le processus de formation de gaz combustible est spécifiquement une fermentation.

**0 014 136**

## Revendications

1. Fermenteur décanteur anaérobie, destiné à l'épuration d'eaux résiduaires notamment de l'industrie du sucre avec récupération des gaz de fermentation constitués en majeure partie de méthane combustible, et comprenant un bassin creusé dans le sol avec une peau d'étanchéité, en forme de pyramide tronquée avec des parois s'évasant vers le haut et un fond sensiblement plat, et adapté à être sensiblement rempli d'eau résiduaires à épurer, vers le centre du bassin un agitateur rotatif à arbre vertical avec un impulseur au voisinage du fond, une bâche souple étendue dans la zone centrale du bassin, ancrée par sa périphérie sensiblement à mi-hauteur des parois et formant poche de collection des gaz de fermentation avec une tubulure d'évacuation des gaz de fermentation en point haut, et une tuyauterie d'amenée d'eaux résiduaires passant par un réchauffeur et débouchant au voisinage de l'impulseur, fermenteur caractérisé en ce qu'une cloche collectrice métallique, avec des bords immergés et traversés axialement par l'arbre de l'agitateur, se raccorde par ses bords à une ouverture centrale de la bâche, celle-ci étant tendue entre ouverture centrale et points d'ancrage périphériques.

2. Fermenteur selon la revendication 1, caractérisé en ce que ladite cloche comporte une chambre annulaire collectrice ouverte vers le bas, limitée latéralement par des bords intérieur et extérieur, celui-ci formant raccordement avec l'ouverture centrale de la bâche, tandis que le bord intérieur entoure l'axe d'agitateur en définissant avec un voile inférieur une cavité fermée.

3. Fermenteur selon la revendication 2, caractérisé en ce que le bord extérieur de cloche est entouré d'une goulotte annulaire ouverte vers le haut, avec un rebord périphérique formant lame déversante et une canalisation d'évacuation d'eau résiduaire épurée.

4. Fermenteur selon une quelconque des revendications 1 à 3, caractérisé en ce que la périphérie de la bâche est raidie par des tiges passant dans des ourlets et rattachés par des tendeurs aux points d'ancrage, ces derniers étant scellés dans un cadre formé par des poutres étendues horizontalement sensiblement à mi-hauteur des parois.

## Claims

1. An anaerobic settling fermenter for the purification of residual waters in particular in the sugar industry with recovery of the fermentation gases of which the major part comprises combustible methane, and comprising a basin which is dug out in the ground, with a sealing skin, in the form of a truncated pyramid with walls widening out upwardly and a substantially flat bottom, and adapted to be substantially filled with residual water to be purified, towards the centre of the basin, a rotary agitator with a vertical shaft, comprising an impeller in the vicinity of the bottom, a flexible cover which is stretched out in the central region of the basin and which is anchored at its periphery substantially halfway up the walls and which forms a pocket for collecting the fermentation gases, with a pipe for discharge of the fermentation gases at a high point, and a pipe system for supplying residual waters, which passes through a heating means and which opens in the vicinity of the impeller, the fermenter being characterised in that a metal collecting bell member having immersed edge portions, through which the shaft of the agitator axially passes, is connected by means of its edge portions to a central opening in the cover, the cover being stretched between the central opening and the peripheral anchorage points.

2. A fermenter according to claim 1, characterised in that said bell member comprises an annular downwardly open collecting chamber which is delimited laterally by inner and outer edge portions, the outer edge portion forming a connection to the central opening in the cover while the inner edge portion is disposed around the agitator axis, defining with a lower veil means a closed cavity.

3. A fermenter according to claim 2 characterised in that the outer edge portion of the bell member is surrounded by an annular upwardly open channel, with a peripheral rim portion forming an overflow weir, and a conduit for discharge of purified residual water.

4. A fermenter according to any one of claims 1 to 3 characterised in that the periphery of the cover is stiffened by rods extending through hems and connected by tensioners to the anchorage points, the latter being sealed in a frame arrangement formed by means extending horizontally substantially halfway up the walls.

## Ansprüche

1. Anaerobe Gärungs-Dekantiereinrichtung, die bestimmt ist zur Klärung von Abwässern, insbesondere der Zuckerindustrie, mit Rückgewinnung der Gärungsgase, die hauptsächlich aus brennbarem Methan bestehen, enthaltend ein in den Boden eingelassenes Becken mit einer Dichtungshaut und von der Gestalt eines Pyramidenstumpfes mit nach oben sich erweiternden Wandungen und einem im wesentlichen flachen Boden, wobei das Becken geeignet ist, um mit den zu klärenden Abwässern im wesentlichen angefüllt zu werden, mit einem in der Nähe der Beckenmitte angeordneten Rührwerk, das eine senkrechte Welle und ein Rührelement in der Bodennähe aufweist, mit einer in der mittleren Zone des Beckens ausgespannten, geschmeidigen Plane, die an ihrem Umfang im wesentlichen auf halber

5

Höhe der Wandungen verankert ist und eine Sammelkammer zum Sammeln der Gärungsgase mit einer an hochgelegener Stelle angeordneten Rohrleitung zur Ableitung der Gärungsgase bildet, und mit einer über eine Heizeinrichtung verlaufenden und in der Nähe des Rührelementes mündenden Rohrleitung zur Zuführung der Abwässer, wobei die Gärungseinrichtung dadurch gekennzeichnet ist, daß eine axial von der Welle des Rührwerks durchquerte Sammelglocke aus Metall mit eingetauchten Rändern über ihre Ränder an eine mittlere Öffnung der Plane anschließt welche zwischen der mittleren Öffnung und am Umfang angeordneten Verankerungspunkten gespannt ist.

2. Gärungseinrichtung nach Anspruch 1, dadurch gekennzeichnet, daß die Glocke eine ringförmige, nach unten offene Sammelkammer aufweist, die seitlich durch den Innenrand und den Außenrand begrenzt ist, wobei dieser Anschluß zur mittleren Öffnung der Plane bildet, während der innere Rand die Achse des Rührwerks umgibt und mit einer unteren Scheibe einen geschlossenen Hohlraum bildet.

3. Gärungseinrichtung nach Anspruch 2, dadurch gekennzeichnet, daß der äußere Rand der Glocke von einer ringförmigen, nach oben offenen Rinne umgeben ist, die einen Umfangsrand aufweist, welcher eine Überlaufkante und eine Kanalleitung zur Ableitung der geklärten Abwässer bildet.

4. Gärungseinrichtung nach einem der Ansprüche 1 bis 3, dadurch gekennzeichnet, daß der Umfang der Plane durch Stangen versteift ist, die durch Saumteile hindurch gehen und durch Spanner mit den Verankerungspunkten verbunden sind, wobei letztere in einen Rahmen eingegossen sind, der durch im wesentlichen auf halber Höhe der Wandungen horizontal verlaufende Träger gebildet ist.

FIG. 1

FIG. 2

FIG. 3

FIG. 4